# EUROPEAN PATENT APPLICATION

(11) **EP 4 755 392 A1**
(43) Date of publication of application: **10.06.2026**
(21) Application number: 24851760.9
(22) Date of filing: 02.08.2024
(51) Int. Cl.: A61K 38/06, A23L 33/145, A23L 33/18, A61K 36/062, A61P 17/16

(54) **ORAL PREPARATION FOR IMPROVING MINIMAL ERYTHEMA DOSE**

(30) Priority: 04.08.2023 JP 2023127821
(71) Applicant: Mitsubishi Corporation Life Sciences Limited, Tokyo 100-0006 (JP)
(72) Inventor: SAKUMA Ayako, Tokyo 100-0006 (JP); SAKURAI Takanobu, Tokyo 100-0006 (JP); URAKAMI Toyoshi, Tokyo 100-0006 (JP)
(74) Representative: Müller-Boré & Partner Patentanwälte PartG mbB
(86) International application number: PCT/JP2024/027647
(87) International publication number: WO 2025/033335

(57) **Abstract**

[Problem] An object of the present invention is to suppress the occurrence of erythema due to ultraviolet irradiation, by using glutathione.

[Solution] According to the present invention, by orally ingesting glutathione at a low dose of 100 mg/day or less, the numerical value of the ultraviolet irradiation level (minimal erythema dose: MED) necessary for the occurrence of erythema can be improved, and the occurrence of erythema due to ultraviolet irradiation can be suppressed. According to the present invention, the tolerance for ultraviolet exposure can be improved, and pigmentation can be suppressed.

## Description

### Technical Field

The present invention relates to a glutathione-containing composition suitably used for improving a minimal erythema dose with respect to ultraviolet irradiation, and a method for improving a minimal erythema dose using the composition.

### Background Art

Although the mechanism of occurrence of pigmentation of the skin, spots, freckles, and the like is unclear, one of causes is considered to be that the melanin pigment is formed due to hormonal abnormalities or stimulation of ultraviolet rays, and this is deposited in the skin. Tyrosinase is an enzyme that catalyzes oxidation of tyrosine, dopa, or the like, and an inhibitor of tyrosinase activity is used as a skin whitening agent for cosmetics or the like or an anti-browning agent for foods by utilizing suppression of generation of the melanin pigment by inhibiting the activity of tyrosinase.

Glutathione is a tripeptide composed of amino acids such as cysteine, glutamic acid, and glycine, and is known as a drug for improving the detoxification action of the liver. A function of improving nonalcoholic fatty liver by ingesting glutathione is known, and glutathione also has a function as a rich taste substance, and is used for food materials, medicines, and the like.

When glutathione is orally ingested, generally, the glutathione is often ingested at 100 mg/day to 300 mg/day, and in Non Patent Literature 1, it is reported that the blood glutathione amount increased by 17% after 6 months by ingesting 250 mg or more. In Non Patent Literature 2, 500 mg/day of glutathione is ingested for 4 weeks, and the effect on melanin of the skin is observed.

As for glutathione, a method for improving the skin condition in combination with other materials has been reported. In Patent Literature 1, it has been reported that the activity of tyrosinase is inhibited by α-lipoic acid and reduced glutathione.

However, redness (erythema) caused on the skin by ultraviolet irradiation has not been reported, and a method for suppressing erythema due to ultraviolet irradiation has been required.

### Citation List

### Patent Literature

Patent Literature 1: JP 2009-51768 A

### Non Patent Literatures

Non Patent Literature 1: Richie JP Jr, Nichenametla S, Neidig W, et al. Randomized controlled trial of oral glutathione supplementation on body stores of glutathione. Eur J Nutr. 2015;54(2):251-263.
Non Patent Literature 2: Arjinpathana, Nutthavuth, Glutathione as an oral whitening agent: A randomized, double-blind, placebo-controlled study. Journal of Dermatological Treatment 2012; 23(2): 97-102

### Summary of Invention

### Technical Problem

An object of the present invention is to suppress the occurrence of erythema due to ultraviolet irradiation, by using glutathione.

### Solution to Problem

The present inventors have conducted intensive studies to solve the above problems, and as a result, have found that by orally ingesting a low dose of glutathione, the occurrence of erythema due to ultraviolet irradiation can be suppressed and the value of the minimal erythema dose can be improved, thereby completing the present invention.

That is, the present invention relates to the following first to fifth aspects.

The first aspect is an oral preparation for improving a minimal erythema dose of skin, containing glutathione as an active ingredient.

The second aspect is the oral preparation for improving a minimal erythema dose of skin of the first aspect, in which a glutathione content is 100 mg or less.

The third aspect is the oral preparation for improving a minimal erythema dose of skin of the first or second aspect, in which the glutathione is glutathione contained in a yeast extract.

The fourth aspect is a method for improving a minimal erythema dose of skin, including orally ingesting glutathione at 100 mg/day or less.

The fifth aspect is the method for improving a minimal erythema dose of skin according to claim 4, in which the glutathione is glutathione contained in a yeast extract.

### Advantageous Effects of Invention

According to the present invention, by orally ingesting glutathione at a low dose of 100 mg/day or less, the occurrence of erythema due to ultraviolet irradiation can be suppressed, and by improving the ultraviolet irradiation level (minimal erythema dose) necessary for the occurrence of erythema, the tolerance for the ultraviolet exposure can be improved.

### Description of Embodiments

Erythema in the present invention refers to redness generated on the skin by ultraviolet irradiation. The ultraviolet irradiation level necessary for the occurrence of erythema is called a minimal erythema dose (MED), and an increase in the numerical value of MED means that the ultraviolet irradiation level necessary for the occurrence of erythema increases. The increase in the numerical value of MED by ingesting glutathione means that glutathione suppresses the occurrence of erythema due to ultraviolet irradiation.

The ultraviolet ray capable of suppressing the occurrence of erythema by glutathione refers to an electromagnetic wave of 10 to 400 nm, and more preferably an electromagnetic wave in a wavelength range of 280 to 320 nm.

The measurement of MED is performed by the following method using a solar simulator. 12 test sections are provided on the back of a subject, the irradiation intensity of the first test section having the weakest irradiation intensity is set to 11.57 mJ/cm^2, and the irradiation intensity of the second adjacent test section is increased by 15% from the irradiation intensity of the first test section. Similarly, the irradiation intensity up to the 12th test section is set as the irradiation intensity in which the irradiation intensity of the n-th test section is increased by 15% as compared with the (n-1)-th test section, and the irradiation intensity of the 12th test section having the strongest irradiation intensity is set to 53.87 mJ/cm^2. The ultraviolet irradiation time is set to 38 seconds. One day after the ultraviolet irradiation by the solar simulator, the presence or absence of erythema at the ultraviolet irradiated site is visually confirmed by a doctor, and the irradiation intensity of the test section having the weakest irradiation intensity among the test sections in which the occurrence of erythema is observed is taken as the MED of the subject.

Erythema of the skin is derived from pigmentation, and thus the degree of pigmentation can be evaluated by L* value. This L* value indicates that the pigmentation is suppressed as the numerical value is higher, that is, erythema is suppressed, and the L* value can be measured using a spectrophotometer.

The L* value refers to a difference in L* value between the irradiated site and the non-irradiated site 7 days after irradiation with ultraviolet light at an irradiation intensity 1.5 times the MED of the subject.

The glutathione described in the present invention refers to reduced glutathione or oxidized glutathione, or a mixture thereof. Reduced glutathione refers to a tripeptide having the γ-L-Glu-L-Cys-Gly structure, and oxidized glutathione refers to a molecule in which two molecules of reduced glutathione are linked by an S-S bond. The form of glutathione may be any form as long as it contains glutathione as an active ingredient.

The glutathione of the present invention is not particularly limited as long as it is obtained by a method that can be used as a medicine or food. Usually, there are a method of obtaining glutathione from a microorganism such as yeast (JP S59-151894 A), a method for producing glutathione by synthesis using an enzyme or the like (JP 2020-72 A), a method for producing glutathione by a chemical synthesis method (Bull. Chem., Soc. Jpn., 1980, vol. 53, p. 2529), and the like, and glutathione used in the present invention is not limited. Glutathione and oxidized glutathione can be purchased, and may be purchased as long as they can be used as foods, medicines, and the like.

The glutathione content in the composition of the present invention is arbitrary, but since the intake amount per day is 100 mg or less, the content in the composition is also preferably 100 mg or less. When the composition of the present invention has, for example, a tablet shape, the content per tablet may be 100 mg, or for example, the content per tablet may be 20 mg, and five tablets may be used as the composition of the present application. The same applies to granules, liquid preparations, and the like.

The glutathione used in the composition containing glutathione of the present invention is not particularly limited as long as it is in a form containing glutathione, such as a purified product, a crude purified product, or a mixture. The mixture may be an extract containing glutathione such as a yeast extract. Examples of the yeast containing glutathione include "HITHION COBO MG" (Mitsubishi Corporation Life Sciences Limited), and examples of the yeast extract containing glutathione include "HITHION EXTRACT YH" (Mitsubishi Corporation Life Sciences Limited).

The method of measuring the glutathione content in the mixture such as a yeast extract is performed in the present invention by using high performance liquid chromatography (HPLC). Measurement conditions are listed below.
· Column: Mightysil RP-18GP (manufactured by KANTO CHEMICAL CO., INC.) 4.6 mm-150 cm
· Mobile phase: 5% diammonium hydrogen phosphate (adjusted to pH 3.2 with phosphoric acid)
· Measurement wavelength: 210 nm

Quantification of total glutathione of both of GSH and GSSG is measured according to the method of Tietze ("Analytical Biochemistry", vol. 27, p. 502, 1969). Note that the glutathione content of the present invention means the total glutathione content of both the reduced form and the oxidized form.

In the present invention, glutathione has an effect even when it is a purified product as long as it is ingested at a low dose, but a glutathione-containing yeast extract is more preferable. As the yeast extract, it is particularly preferable to use a glutathione-rich yeast extract. A yeast extract containing glutathione in an amount of 8 wt% or more is particularly preferable, and a yeast extract containing glutathione in an amount of 10 wt% or more is more preferable. An enzyme extract containing glutathione in an amount of 15 wt% or more is particularly preferable.

In the composition of the present invention, other substances can be added as long as the effect of the present invention is not impaired. As those that can be added, normal supplements, feed, excipients added to medicines, various vitamins, inorganic substances such as calcium, magnesium, iron, and zinc, organic acids such as lactic acid, citric acid, and malic acid, organic substances such as proteins, lactic acid bacteria extracts, polyphenols, and plant extracts, and the like may be appropriately added. An acidulant, a sweetener, a flavor, a stabilizer, and the like, which are added to general foods, can also be added.

The method for administering the present invention product is not particularly limited, and can be exemplified by oral administration, or non-oral administration such as intravenous, intraperitoneal or subcutaneous administration. Specifically, either oral agent such as tablets, powders, granules, pills, suspensions, emulsions, infusions and decoctions, capsules, syrups, liquid, elixir, extract, tinctures and fluidextracts; or non-oral agent such as injections, drip preparations, creams, and suppositories, may be used. As a method for adjusting these tablets and the like, a conventional method can be employed, and there is no limitation in the present invention.

The dosage of the present invention is effective at low doses. The intake amount and the number of administrations in the case of administration to a human vary depending on the administration form, the age and body weight of the subject, and the like, but glutathione is ingested so as to be 100 mg or less per day for an adult. Usually, it is ingested once or several times a day so as to be 10 mg to 100 mg, preferably 20 mg to 80 mg, and particularly preferably 30 mg to 50 mg. When a yeast extract containing glutathione is used, the glutathione content in the yeast extract is measured, and the intake amount is adjusted so as to be the dose of this paragraph.

The composition of the present invention can be ingested not only as a medicine but also as a functional food or a nutritional supplement, and in that case, the composition may be ingested so as to have the intake amount at the preceding stage.

The method for improving the minimal erythema dose of the present invention can be known by how much the irradiation intensity of MED is improved by ingesting glutathione by the ingestion method of glutathione at the preceding stage.

Similarly, the method for improving the L* value of the present invention can be known from the difference in the L value between the irradiated site and the non-irradiated site 7 days after the skin is irradiated with ultraviolet rays having an irradiation intensity 1.5 times the MED.

### Examples

Hereinafter, the present invention will be specifically described with reference to Examples, but the present invention is not limited thereto.

### <Test Method>

In a randomized, placebo-controlled, double-blind, parallel-group comparative trial for healthy adults of 30 years old or more and 59 years old or less, by ingesting the test food for 5 weeks, the influence on the occurrence of erythema due to ultraviolet irradiation was verified.

### <Test Food>

· A yeast extract A is a yeast extract containing glutathione in an amount of 15 wt%, and the glutathione content in a test food A is 40 mg of glutathione.
· Since maltitol (manufactured by Mitsubishi Corporation Life Sciences Limited) was used as a control food, the glutathione content in the control food is 0 mg.

### <Test Period>

March to April, 2023.

### <Dose>

The test food group orally ingests the test food A, and the intake amount of each component per day is 40 mg of glutathione.

The placebo group orally ingests the control food, and components other than glutathione are the same as those of the test food A.

### <Measurement of MED>

The measurement of the MED (minimal erythema dose) was performed as follows. 12 test sections were provided on the back of a subject, the irradiation intensity of the first test section having the weakest irradiation intensity was set to 11.57 mJ/cm^2, and the irradiation intensity of the second adjacent test section was increased by 15% from the irradiation intensity of the first test section. Similarly, the irradiation intensity up to the 12th test section was set as the irradiation intensity in which the irradiation intensity of the n-th test section was increased by 15% as compared with the (n-1)-th test section, the irradiation intensity of the 12th test section having the strongest irradiation intensity was set to 53.87 mJ/cm^2, and ultraviolet irradiation (wavelength: 290 to 320 nm) was performed using a solar simulator. The ultraviolet irradiation time was set to 38 seconds. One day after the ultraviolet irradiation, the presence or absence of erythema was visually confirmed by a doctor, and the irradiation intensity of the test section having the lowest irradiation intensity among the test sections in which the occurrence of erythema was observed was taken as the MED before the test food A or the control food was ingested by the subject (MED before ingestion).

After the MED measurement, the ingestion of the test food A or the control food was started, and 28 days after the start of the ingestion, the MED measurement was performed as described above to obtain the MED after the ingestion of the test food A or the control food (MED after ingestion). Note that the ingestion of the test food A or the control food was continued until 36 days after the start of the ingestion.

The average value and standard error of MED of each of the test food A ingestion group (test food group) and the control food ingestion group (placebo group) were calculated, and a significant difference test was performed by Wilcoxon signed-rank test. The amount of change in the MED before and after ingestion of each group was calculated, and a significant difference test was performed by Mann-Whitney U test.

### <Measurement Results of MED>

As the value of MED is larger, it means that the irradiation intensity of ultraviolet light required to generate erythema is stronger. The measurement results of MED are shown in Table 1 as the average value ± the standard error. As a result, in the MED after ingestion, the test food group had a MED of 37.29 ± 1.06, whereas the placebo group had a MED of 34.25 ± 0.99. On the other hand, no significant difference was observed between both groups before ingestion.

When comparing the MED before and after the ingestion of the test food group, the difference was significant (*p <* 0.001), but there was no significant difference in MED before and after the ingestion of the placebo group (*p >* 0.1).

When the amount of change in MED before and after ingestion was compared between the groups, the difference was significant (p < 0.05).

**[Table 1]**

| | MED before ingestion | MED after ingestion | Amount of change in MED before and after ingestion |
|---|---|---|---|
| Test food group | 33.94 ± 0.71 | 37.29 ± 1.06 | 3.35 ± 0.79 |
| Placebo group | 33.77 ± 0.64 | 34.25 ± 0.99 | 0.48 ± 0.66 |

### <Measurement Test of L* Value>

Irradiation was performed with an ultraviolet ray (wavelength: 290 to 320 nm) having an irradiation intensity 1.5 times the MED before ingestion of each subject measured in the MED measurement test, and after 7 days after irradiation, the L* value of the irradiated site was measured using a spectrophotometer (CM-26d; KONICA MINOLTA JAPAN, INC.). At the same time, the L* value of the non-irradiated site was also measured.

After 28 days from the start of ingestion of the test food A or the control food, ultraviolet irradiation was performed at an irradiation intensity 1.5 times the MED before ingestion as described above, and after 7 days, the L* value of the irradiated site (the L* value after ingestion) was measured. At the same time, the L* value of the non-irradiated site was also measured.

The difference between the L* value of the irradiated site and the L* value of the non-irradiated site, which were measured at the same time, was calculated as the ΔL* value of the subject, and the ΔL* values before and after the ingestion of the test food A or the control food were calculated. The difference between the ΔL* value before ingestion and the ΔL* value after ingestion was taken as the amount of change in the ΔL* value.

Regarding the ΔL* values before and after the ingestion of the test food A or the control food in each group, a significant difference test was performed by paired t-test. Regarding the amount of change in the ΔL* value between the groups, a significant difference test was performed by unpaired t-test.

### <Measurement Results of ΔL* Value>

The L* value indicates the degree of pigmentation due to ultraviolet irradiation, and as the numerical value is higher, it means that the pigmentation is suppressed. The results of the L* value are shown in Table 2. First, when comparing cases before and after the test food ingestion, it was revealed that the L* value was significantly improved (p < 0.001). On the other hand, there was no improvement in the L* value in the control food ingestion group.

When the amount of change in the L* value was compared between the groups, it was revealed that the test food group can significantly improve the L* value as compared with the placebo group (p < 0.001).

As for the L* value before and after ingestion, a value obtained by subtracting the L* value of the non-irradiated site from the L* value of the irradiated site was taken as a ΔL* value, the ΔL* value was calculated from the L* value of each group, and the amount of change in the ΔL* value before and after ingestion was calculated. As the amount of change in the ΔL* value is larger, it is shown that the pigmentation is more suppressed. The ΔL* value of the test food group was 1.003, the ΔL* value of the placebo group was -0.510, and the difference was significant (p < 0.001).

**[Table 2]**

| | L* value before ingestion | L* value after ingestion | Amount of change in L* value before and after ingestion |
|---|---|---|---|
| Test food group | 61.33 ± 0.35 | 62.44 ± 0.33 | 1.316 ± 0.241 |
| Placebo group | 60.79 ± 0.40 | 60.00 ± 0.43 | -0.792 ± 0.197 |

From the above. it was shown that by ingesting glutathione at a low dose, the amount of ultraviolet light necessary for the occurrence of erythema can be improved, and by further suppressing pigmentation, the occurrence of erythema due to ultraviolet irradiation can be suppressed.

## Claims

1. An oral preparation for improving a minimal erythema dose of skin, comprising glutathione as an active ingredient.

2. The oral preparation for improving a minimal erythema dose of skin according to claim 1, wherein a glutathione content is 100 mg or less.

3. The oral preparation for improving a minimal erythema dose of skin according to claim 1 or 2, wherein the glutathione is glutathione contained in a yeast extract.

4. A method for improving a minimal erythema dose of skin, comprising orally ingesting glutathione at 100 mg/day or less.

5. The method for improving a minimal erythema dose of skin according to claim 4, wherein the glutathione is glutathione contained in a yeast extract.
